# EUROPÄISCHE PATENTSCHRIFT

(11) **EP 1 395 580 B1**
(45) Veröffentlichungstag und Bekanntmachung des Hinweises auf die Patenterteilung: **15.12.2004**
(21) Anmeldenummer: 02745289.5
(22) Anmeldetag: 10.05.2002
(51) Int. Cl.: C07D 405/06

(54) **VERFAHREN ZUR REINIGUNG VON TRIAZOLYLMETHYL-OXIRANEN**
METHOD FOR THE PURIFICATION OF TRIAZOLYLMETHYLEPOXIDES
PROC D DE PURIFICATION DE TRIAZOLYLM THYLE-OXIRANNES

(30) Priorität: 18.05.2001 DE 10124664
(43) Veröffentlichungstag der Anmeldung: 10.03.2004
(73) Patentinhaber: BASF AKTIENGESELLSCHAFT, 67056 Ludwigshafen (DE)
(72) Erfinder: NOACK, Rainer, 04932 Grossthiemig (DE); KRAFFZIK, Reinhold, 67281 Kirchheim (DE); KOARK, Dietmar, 01987 Schwarzheide (DE)
(86) Internationale Anmeldenummer: PCT/EP2002/005148
(87) Internationale Veröffentlichungsnummer: WO 2002/094817

(56) Entgegenhaltungen:
- EP-A- 0 409 049
- DE-C- 19 618 578
- T. BURGER ET AL: "Synthesis of four 14C-isotopomers of epoxiconazole, a new triazole fungicide" JOURNAL OF LABELLED COMPOUNDS AND RADIOPHARMACEUTICALS, Bd. 38, Nr. 2, 1996, Seiten 173-178, XP008007186 SUSSEX, GB ISSN: 0362-4803
- H. MIYAUCHI ET AL: "Asymmetric synthesis of SM-9164, a biologically active enantiomer of antifungal agent SM-8668" BULLETIN OF THE CHEMICAL SOCIETY OF JAPAN., Bd. 69, Nr. 9, 1996, Seiten 2625-2632, XP002210961 JAPAN PUBLICATIONS TRADING CO. TOKYO., JP ISSN: 0009-2673

## Beschreibung

Die vorliegende Erfindung betrifft ein Verfahren zur Reinigung von Triazolylmethyl-oxiranen der Formel I, 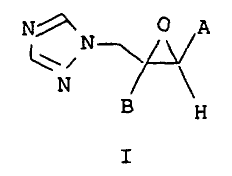 in der A und B gleich oder verschieden sind und unabhängig voneinander C₁-C₄-alkyl, Phenyl-C₁-C₂-alkyl, C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten, ausgewählt aus der Gruppe: Halogen, Nitro, C₁-C₄-Alkyl, C₁-C₄-Alkyloxy, Phenoxy, Amino, C₁-C₂-Halogenalkyl oder Phenylsulfonyl tragen kann, die durch Umsetzung eines Oxirans der Formel II 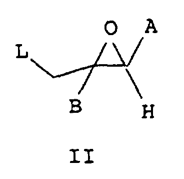 in der A und B die vorgenannte Bedeutung besitzen und L für eine nukleophil austauschbare Abgangsgruppe steht, mit
a) 1,2,4-Triazol (IIIa) und einem Moläquivalent einer anorganischen Base oder
b) 1,2,4-Triazolid der Formel IIIb, 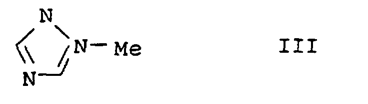 IIIa:Me = Wasserstoff
   IIIb:Me = Alkalimetall
   in der Me für ein Alkalimetallatom oder ein quarternäres Ammoniumion steht, gewonnen werden.

Azolylmethyl-oxirane dienen zur Herstellung von fungiziden Mitteln, insbesondere gegen Getreidekrankheiten.

Aus dem Stand der Technik sind einige Verfahren zur Herstellung von Triazolylmethyl-oxiranen ausgehend von Oxiran II mit Natrium-1,2,4-triazolid bekannt (s. DE-A 39 36 823, EP-A 330132 und EP-A 334 035). Als Lösungsmittel kommen in der Regel Dimethylformamid und N-Methylpyrrolidon zur Anwendung. Die Aufarbeitung der erhaltenen Triazolierungsprodukte erfolgt - wie beispielsweise in der DE-A 39 36 823 beschrieben - durch Fällung mit Wasser und/oder Extraktion.

Die Verfahren des Standes der Technik sind mit einer Reihe von Nachteilen behaftet. Bei der Triazolierung von Verbindungen der Formel II entstehen neben den gewünschten 1- auch 4- substituierte Triazole in Anteilen von 5-25 %.

In Verbindung der Formel I befinden sich darüberhinaus chirale Zentren, so daß im allgemeinen als Diastereomerengemische erhalten werden.

Weiterhin entstehen durch Solvolyse und Ringöffnungsreaktionen eine Reihe von Nebenprodukten, die die Ausbeute verringern und die Isolierung und Reinigung der gewünschten Triazolylmethyloxirane erheblich erschweren.

Zur Reinigung der anfallenden Isomerengemische werden genannt:
- Extraktion (z.B. DE-A 32 18 130, US 4,906,652),
- Fällung (z.B. DE-A 39 36 823),
- Chromatografie (z.B. DE-A 38 06 089),
- Rekristallisation aus Disopropypether (DE-A 39 36 823, US 4,906 652), Methyltert.butylether/n-Hexan (DE-A 38 05 376, EP-A 330 132), Methyltert.butylether (DE-A 37 37 888),

Um die nötige Reinheit der Pflanzenschutzwirkstoffe zu erhalten, muß in der Regel eine Kombination der o.g. Methoden angewandt werden.

Die Reinheit der fungizid wirksamen Isomeren ist überwiegend unter 92 %, nur nach vorbeschriebener komplizierter Aufarbeitung kann man vertretbare Gehalte von über 94 % erreichen.

Wirtschaftliches Verfahren zur Reinigung von Triazolylmethyl-oxiranen der Formel II bis zu Wirkstoffgehalten von über 96 % bei hohen Raum-Zeit-Ausbeuten sind aus den sogenannten Gründen im technischen Maßstab bislang nur beschränkt verfügbar.

Der Erfindung lag deshalb die Aufgabe zugrunde, eine wirtschaftliche Reinigungsmethode für fungizid wirksame Isomere von Azolylmethyl-oxiranen mit dem Ziel hohe Reinheit, hohe Raum-Zeit-Ausbeuten und einfacher Produktisolierung zu finden.

Überraschenderweise konnte ein solches Verfahren zur Reinigung von Triazolyloxiranen dadurch gefunden werden, daß man das gefällte und auf einer Zentrifuge abgetrennte Produkt als Filterkuchen einer kurzzeitigen Wäsche mit einem speziellen Lösungsmittel oder Lösungsmittelmischung unterwirft. Dabei wird ein Lösungsmittel bzw. eine Lösungsmittelmischung eingesetzt, die ein mittleres bis hohes Lösevermögen für die im Filterkuchen enthaltene Stoffe einschließlich des Wertproduktes besitzt. Demgegenüber arbeitet man nach dem Stand der Technik immer mit Lösungsmitteln, die kein oder ein geringes Lösevermögen für die gefällten Produkte besitzen, z.B. mit Wasser.

Die erfindungsgemäßen Lösungsmittel sind in a) und b) genannt.

Ein erfindungsgemäßes Lösungsmittel enthält:
a) die bereits oben genannten für die Triazolierungsreaktionen üblichen dipolar aprotischen Lösungsmittel, wie DMF, DMAA, NMP, TMH, DMSO bzw. Sulfolan und gegebenenfalls
b) ein damit mischbares Cosolvents, z.B. niedrigmolekularen Alkoholen wie Methanol, Ethanol, Isopropanol, tert. Butanol, Glykol, Methylglykol, etc., oder Wasser.

In Mischungen kann zur Einstellung der Löslichkeit auch Wasser eingesetzt werden.

Durch die Anwendung der erfindungsgemäßen Methode können aufgrund von Unterschieden in der Löslichkeit bzw. der Fällungsreihenfolge und in Abhängigkeit von der Verweilzeit die an den Kristalloberflächen befindlichen isomeren Nebenprodukten bzw. andere Nebenprodukte gelöst und aus dem Filterkuchen entfernt werden.

Die Wirkungsweise dieser Kontaktwäsche kann modifiziert werden durch die Wahl und die Zusammensetzung des Lösungsmittelsystems, die Temperatur und die Einwirkzeit.

Im folgenden wird das erfindungsgemäße Verfahren näher erläutert.

Für das erfindungsgemäße Verfahren eignen sich Azolylmethyloxiranen, die aus folgenden Ausgangsmaterialien hergestellt worden sind.
a) Oxirane der Formel II in der A und B gleich oder verschieden sind und unabhängig voneinander C₁-C₄-Alkyl, Phenyl-C₁-C₂-alkyl, C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten. Ausgewählt aus der Gruppe: Halogen, Nitro, C₁-C₄-Alkyl, C₁-C₄-Alkyloxy, Phenoxy, Amino, C₁-C₂-Halogenalkyl oder Phenylsulfonyl tragen kann und L für eine nukleophil substituierbare Abgangsgruppe steht. Die Oxirane lassen sich wie in EP-A 94 564, US 4,906,652, EP-A 330132, EP-A 334 035 und DE-39 36 823 beschrieben herstellen. Bevorzugte Ausgangsmaterialien tragen die folgenden Substituenten, wobei die Bevorzugung jeweils für sich allein oder in Kombination zu sehen ist:

A und B bedeuten vorzugsweise einen durch Halogen, C₁-C₄-Alkyl oder C₁-C₄-Alkyloxy substituierten Phenylrest.

Insbesondere bedeutet A 4-Fluorphenyl und B 2-Chlorphenyl.

L steht für eine nukleophil substituierbare Abgangsgruppe wie beispielsweise Halogenid, Alkylsulfonat, Arylsulfonat oder Alkylsulfat. Vorzugsweise bedeutet L Chlorid, Bromid, Tosylat und insbesondere Mesylat.

Als Triazolide eignen sich Verbindungen der Formel IIIb, 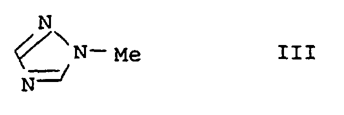 in der Me für ein Alkalimetall oder ein quartäres Ammonium, insbesondere für Natrium oder Kalium steht.

Geeignete Basen zum Einsatz in der Verfahrensvariante a) bzw. Herstellung von Triazolid IIIb (Verfahrensvariante b) sind Alkalihydroxide wie beispielsweise Natrium- oder Kaliumhydroxid, quartäre Ammoniumhydroxide, Alkalimetallcarbonate wie beispielsweise Natrium-, Kalium- oder Natriumhydrogencarbonat, Alkalimetallakoholate wie beispielsweise Natriummethylat oder Kaliumtert.-butylat, Alkalicarboxylat wie beispielsweise Kaliumformylat oder Natriumacetat, Alkalimetallphenolat wie beispielsweise Natriumphenolat, oder Hydride wie Natriumhydrid oder Natriumboranat.

Die bei der Triazolierung erhältliche Reaktionsmischung wird mit Wasser ausgefällt und vorzugsweise in Portionen auf eine Schälzentrifuge gegeben. Der nach der Abschleudern der Mutterlauge verbleibende Filterkuchen wird anschießend der erfindungsgemäßen Wäsche mit einem der bereits oben angegebenen Lösungsmittel oder Lösungsmittelmischungen unterzogen.

Bevorzugt sind Lösungsmittel wie N-Methylpyrrolidon, Dimethylformamid, Methanol, tert.-Butanol, und Mischungen davon. Weiterhin bevorzugt ist das Lösungsmittel: Dimethylformamid/Wasser.

Besonders bevorzugt ist die Mischung Methanol/ Dimethylformamid.

Lösungsmittelmischungen enthalten vorzugsweise 60 bis 90% eines C1-C6-Alkohols und 10 bis 40% eines Amids wie N-Methylpyrrolidon oder Dimethylformamid.

Die aufzugebenden Mengen an Lösungsmitteln liegen zwischen 0,1 und 1,0 l/kg Filterkuchen.

Die Einwirkzeit der Lösungsmittel sollte bei 0-20 s liegen. Längere Einwirkzeiten sind nicht empfehlenswert, da es dann zur partiellen Auflösung des Filterkuchen kommen kann. Gleichfalls sollten Temperaturen von 30°C nicht überschritten werden.

Das erfindungsgemäße Verfahren wird durch die folgenden Beispiele erläutert.

### Durchführungsbeispiele

### Beispiel 1

In einem 10 m³-Reaktor wurden zu 1236 kg 2-(Methansulfonyloxymethyl)-2-(4-fluorphenyl)-3-(2-chlorphenyl)oxiran (MES/cis-trans 5:95), gelöst in 2664 kg Dimethylformamid, 320 kg 1,2,4-Triazol und 750 kg 30%ige Natriummethanolatlösung hinzugefügt und 5 h bei 75°C gerührt. Nach komplettem Umsatz des MES wurde auf 35°C gekühlt. Man fällte mit 820 kg 25°C warmen Wasser mit Zugabegeschwindigkeit von anfangs 300 l/h, später mit 800 l/h. Die erhaltene Kristallmaische wurde in einer Schälzentrifuge von der Mutterlauge getrennt, der Filterkuchen mit Dimethylformamid/Wasser (70:30, ca. 0,7 l/kg Filterkuchen) gewaschen und anschließend der üblichen Wasserwäsche unterzogen (ca. 5 l/kg Filterkuchen) und trockengeschleudert. Man erhielt ca. 800 kg eines Produktes mit folgenden analytischen Daten:
trans-Epoxiconazol = 96,6 %, sym.-Epoxiconazol = 2,21 %, cis Epoxiconazol < 0,01 % EPA(2-Hydroxy-methyl-2-(fluorphenyl)-3-(2-chlorphenyl)-oxiran) = 0,17 %

### Beispiel 2

In einem 2,5 m³-Reaktor wurden zu 311 kg MES(cis-trans 5:95), gelöst in 675 kg DMF 75 kg Triazol und 187 kg Natriummethanolat hinzugefügt und 5 h bei 65°C gerührt. Nach komplettem Umsatz des MES wird auf 35°C gekühlt. Man fällte mit 25°C warmen Wasser mit Zugabegeschwindigkeit von anfangs 300 l/h, später mit 80 l/h. Die erhaltene Kristallmaische wurde an einer Schälzentrifuge von der Mutterlauge getrennt und auf der Zentrifuge mit Methanol/DMF (75:25, ca. 0,2 l/kg Filterkuchen) gewaschen. Nach der Nachwäsche mit Wasser und Trockenschleudern erhielt man 200 kg eines Produktes, das getrocknet folgenden analytischen Daten aufwies:
trans-Epoxiconazol = 97,50 %, sym.-Epoxiconazol = 2,23 %, cis Epoxiconazol = 0,06 % EPA(2-Hydroxy-methyl-2-(fluorphenyl)-3-(2-chlorphenyl)-oxiran) = 0,08 %

### Vergleichsbeispiel

Die Umsetzung und Reinigung erfolgte analog Beispiel 2, man verzichtete aber auf die erfindungsgemäße Wäsche mit Methanol/DMF. Somit erhielt man ein Produkt mit folgenden analytischen Daten nach Trocknung:
trans-Epoxiconazol = 92,8 %, sym.-Epoxiconazol = 2,84 %, cis Epoxiconazol = 0,64 % EPA(2-Hydroxy-methyl-2-(fluorphenyl)-3-(2-chlorphenyl)-oxiran) = 0,32 %

### Beispiel 3

Man verfuhr wie im Beispiel 1, verwendete aber zur Wäsche Dimethylformamid (0,18 l/kg Filterkuchen). Nach der Trocknung erhält man ein Produkt mit einem Gehalt von 96,8 % trans-Epoxiconazol. Der Vergleich ohne Zwischenwäsche erreicht ein Gehalt von 92,5 %.

## Patentansprüche

1. Verfahren zur Reinigung von Triazolylmethyloxiran-Isomeren der allgemeinen Formel I 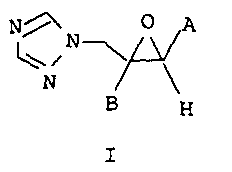 in der A und B gleich oder verschieden sind und unabhängig voneinander C₁-C₄-Alkyl, Phenyl-C₁-C₂-alkyl, C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten, ausgewählt aus der Gruppe: Halogen, Nitro, C₁-C₄-Alkyl, C₁-C₄-Alkyloxy, Phenoxy, Amino, C₁-C₂-Halogenalkyl oder Phenylsulfonyl tragen kann, die durch Umsetzung eines Oxirans der Formel II 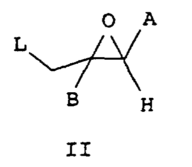 in der A und B die vorgenannte Bedeutung besitzen und L für eine nukleophil austauschbare Abgangsgruppe steht, mit
a) 1,2,4-Triazol (IIIa) und einem Moläquivalent einer anorganischen Base oder
b) 1,2,4-Triazolid der Formel IIIb, 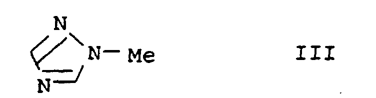 IIIa: Me = Wasserstoff
IIIb:Me = Alkalimetall
in der Me für ein Alkalimetallatom oder ein quarternäres Ammoniumion steht, gewonnen werden, **dadurch gekennzeichnet, daß** man das nach der Triazolierung, Fällung und Zentrifugieren erhaltene Isomerengemisch einer Wäsche mit einem Lösungsmittel enthaltend
a) ein dipolares aprotisches Amid, Sulfoxid, Urethan oder einen Harnstoff, und gegebenenfalls
b) einen C₁-C₆-Alkohol, ein Glykol oder Wasser,
unterwirft.

2. Verfahren gemäß Anspruch 1, **dadurch gekennzeichnet, daß** das Lösungsmittel:
a) Dimethylformamid, Dimethylacetamid oder N-Methylpyrrolidon und
b) Methanol, Ethanol oder tert. Butanol
enthält.

3. Verfahren gemäß Anspruch 1 und 2, **dadurch gekennzeichnet, daß** das Lösungsmittelmischung Dimethylformamid und Methanol enthält.

4. Verfahren gemäß Anspruch 1, **dadurch gekennzeichnet, daß** die Lösungsmittel Dimethylformamid und Wasser enthält.

5. Verfahren gemäß Anspruch 1 bis 4, **dadurch gekennzeichnet, daß** die Wäsche bei Temperaturen zwischen 10 und 60°C durchgeführt wird.

6. Verfahren gemäß Anspruch 1 bis 5, **dadurch gekennzeichnet, daß** die Wäsche mit Lösungsmittelmengen von 0,1-2,0 l/kg Filterkuchen durchgeführt wird.

7. Verfahren gemäß Anspruch 1 bis 6, **dadurch gekennzeichnet, daß** in Formel I die Substituenten A = 4-Fluorphenyl und B = 2-Chlorphenyl bedeuten.

## Claims

1. A process for the purification of triazolylmethyloxirane isomers of the formula I 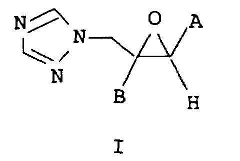 in which A and B are identical or different and, independently of one another, are C₁-C₄-alkyl, phenyl-C₁-C₂-alkyl, C₃-C₆-cycloalkyl, C₃-C₆-cycloalkenyl, tetrahydropyranyl, tetrahydrofuranyl, dioxanyl or phenyl, where the phenyl radical may carry one to three substituents chosen from the group: halogen, nitro, C₁-C₄-alkyl, C₁-C₄-alkyloxy, phenoxy, amino, C₁-C₂-haloalkyl or phenylsulfonyl, which are obtained by reacting an oxirane of the formula II 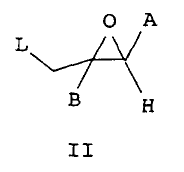 in which A and B have the meanings given above and L is a nucleophilically substitutable leaving group, with
a) 1,2,4-triazole (IIIa) and one mole equivalent of an inorganic base or
b) 1,2,4-triazolide of the formula IIIb, 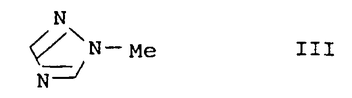 IIIa: Me = hydrogen
IIIb:Me = alkali metal
in which Me is an alkali metal atom or a quaternary ammonium ion, which comprises subjecting the isomer mixture obtained after the triazolation, precipitation and centrifugation to washing with a solvent comprising
a) a dipolar aprotic amide, sulfoxide, urethane or a urea, and optionally
b) a C₁-C₆-alcohol, a glycol or water.

2. A process as claimed in claim 1, wherein the solvent comprises
a) dimethylformamide, dimethylacetamide or N-methylpyrrolidone and
b) methanol, ethanol or tert-butanol.

3. A process as claimed in claim 1 or 2, wherein the solvent mixture comprises dimethylformamide and methanol.

4. A process as claimed in claim 1, wherein the solvent comprises dimethylformamide and water.

5. A process as claimed in claims 1 to 4, wherein the washing is carried out at temperatures between 10 and 60°C.

6. A process as claimed in claims 1 to 5, wherein the washing is carried out with solvent amounts of 0.1-2.0 l/kg of filter cake.

7. A process as claimed in claims 1 to 6, wherein, in formula I, the substituents A = 4-fluorophenyl and B = 2-chlorophenyl.

## Revendications

1. Procédé pour la purification d'isomères de triazolylméthyloxiranne répondant à la formule générale I 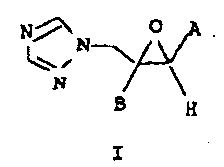 dans laquelle A et B sont identiques ou différents et représentent, indépendamment l'un de l'autre, un groupe alkyle en C₁-C₄, un groupe phénylalkyle en C₁-C₂, un groupe cycloalkyle en C₃-C₆, un groupe cycloalcényle en C₃-C₆, un groupe tétrahydropyranyle, un groupe tétrahydrofuranyle, un groupe dioxanyle ou un groupe phényle, le radical phényle pouvant porter de un à trois substituants choisis parmi le groupe comprenant un atome d'halogène, un groupe nitro, un groupe alkyle en C₁-C₄, un groupe alkyl(en C₁-C₄)oxy, un groupe phénoxy, un groupe amino, un groupe halogénoalkyle en C₁-C₂ ou un groupe phénylsulfonyle, que l'on obtient par la mise en réaction d'un oxiranne répondant la formule II 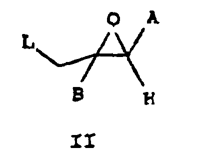 dans laquelle A et B ont la signification mentionnée ci-dessus et L représente un groupe de départ qui peut être soumis à un échange nucléophile, avec
a) du 1,2,4-triazole (IIIa) et un équivalent molaire d'une base inorganique ou
b) du 1,2,4-triazolide répondant à la formule IIIb, 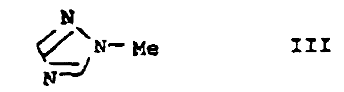 IIIa : Me = un atome d'hydrogène
IIIb : Me = un métal alcalin
dans laquelle Me représente un atome de métal alcalin ou un ion d'ammonium quaternaire, **caractérisé en ce qu'**on soumet le mélange d'isomères obtenu après la triazolation, la précipitation et la centrifugation, à un lavage avec un solvant contenant
a) un amide aprotique dipolaire, un sulfoxyde, un uréthane ou une urée, et éventuellement
b) un alcool en C₁-C₆, un glycol ou de l'eau.

2. Procédé selon la revendication 1, **caractérisé en ce que** le solvant contient
a) du diméthylformamide, du diméthylacétamide ou de la N-méthylpyrrolidone et
b) du méthanol, de l'éthanol ou du tert-butanol.

3. Procédé selon les revendications 1 et 2, **caractérisé en ce que** le mélange de solvants contient du diméthylformamide et du méthanol.

4. Procédé selon la revendication 1, **caractérisé en ce que** les solvants contiennent du diméthylformamide et de l'eau.

5. Procédé selon les revendications 1 à 4, **caractérisé en ce qu'**on effectue le lavage à des températures entre 10 et 60 °C.

6. Procédé selon les revendications 1 à 5, **caractérisé en ce qu'**on effectue le lavage avec des quantités de solvants de 0,1 à 2,0 l/kg du gâteau de filtre.

7. Procédé selon les revendications 1 à 6, **caractérisé en ce que**, dans la formule I, les substituants A et B représentent respectivement un groupe 4-fluorophényle et un groupe 2-chlorophényle.
